# FASCICULE DE BREVET EUROPEEN

(11) **EP 0 586 313 B1**
(45) Date de publication et mention de la délivrance du brevet: **27.12.1996**
(21) Numéro de dépôt: 93420349.8
(22) Date de dépôt: 26.08.1993
(51) Int. Cl.: A61B 17/064

(54) **Agrafe chirurgicale et appareil pour son impaction**
Chirurgische Klammer und Gerät zur deren Anwendung
Surgical staple and device for its impaction

(30) Priorité: 02.09.1992 FR 9210722
(43) Date de publication de la demande: 09.03.1994
(73) Titulaire: ORTHOMED Sarl, 21160 Marsannay la Côte (FR)
(72) Inventeur: Comte Georges, F-21000 Dijon (FR)
(74) Mandataire: Dupuis, François

(56) Documents cités:
- FR-A- 2 590 793
- FR-A- 2 603 794
- GB-A- 2 118 662
- US-A- 4 263 903
- US-A- 4 592 346

## Description

L'invention concerne plus particulièrement une agrafe chirurgicale du type de celle utilisée en ostéosynthèse et pour la fixation des ligaments naturels ou artificiels. L'invention concerne également l'appareil permettant l'impaction et l'extraction de cette agrafe.

D'une manière parfaitement connue pour un homme du métier, ces agrafes chirurgicales comprennent deux branches parallèles d'ancrage réunies par une partie commune rectiligne, de sorte que l'agrafe a une section très sensiblement en forme de U renversé. Les branches de l'agrafe sont effilées à leurs extrémités pour faciliter l'impaction.

On connait également des appareils permettant le positionnement et l'enfoncement de l'agrafe par rapport au foyer d'ostéotomie et également son extraction. Cet état de la technique peut par exemple être illustré par l'enseignement du brevet US-A-4263903. Pour l'essentiel, l'agrafe définie dans ce brevet présente une partie commune de section transversale en forme de queue d'arronde destinée à être engagée dans une partie correspondante que présente un appareil impacteur. L'appareil présente une partie active constituée par deux mors, faisant office de pince, assujettis à un organe de manoeuvre formé en bout d'un manche de préhension. Les deux mors délimitent, en combinaison, une empreinte correspondant à la section en queue d'arronde de la partie commune de l'agrafe.

Si cette solution donne satisfaction pour l'impaction et l'extraction de l'agrafe, il apparait que la section en queue d'arronde de cette dernière constitue des parties saillantes générant des risques de traumatismes pour les tissus. Cet inconvénient est d'autant plus important lorsque l'agrafe affleure la peau. Or de telles situations sont des plus fréquentes.

Pour tenter de remédier à ces incovénients, on a proposé une agrafe dont la partie commune destinée à coopérer avec l'appareil impacteur présente une section sans aucune aspérité. Cet état de la technique peut être illustré par l'enseignement du brevet FR-A-2603 794. L'agrafe définie dans ce brevet présente une partie commune, de section transversale arrondie sur plusieurs rayons de courbure de valeur décroissante depuis la face inférieure de ladite partie d'où partent les branches d'ancrage, jusqu'à sa face supérieure. Il apparait donc que la face inférieure de cette partie commune est reliée à la face supérieure par un large rayon de courbure suivant très sensiblement un quart de cercle.

L'appareil impacteur, du type de celui défini dans le brevet US précité, présente une partie active constituant une pince dont les branches définissent une empreinte correspondant à la section transversale de la partie commune de l'agrafe c'est-à-dire avec un large rayon de courbure au niveau notamment de son extrémité libre. Il en résulte des risques de déviation de l'agrafe au moment de son impaction lorsque l'on exerce un effort de frappe sur l'appareil. En effet, compte tenu des bords arrondis de la partie commune de l'agrafe au niveau de sa face inférieure et du profil également arrondi de l'empreinte des pinces recevant l'agrafe, le blocage angulaire de cette dernière n'est pas assuré de manière suffisante.

L'invention s'est fixée pour but de remédier à ces inconvénients de manière simple, sûre et efficace.

Le double problème que se propose de résoudre l'invention, est d'obtenir un blocage angulaire de l'agrafe dans l'appareil impacteur en évitant tous risques de déplacement angulaire tout en ayant pour objectif de n'avoir aucune partie saillante au niveau de la partie commune de raccordement de l'agrafe évitant ainsi tout risque de blessures traumatiques après impaction.

Pour résoudre un tel problème, il a été conçu et mis au point une agrafe du type de celle présentant deux branches parallèles d'ancrage réunies par une partie commune rectiligne de section transversale méplate et présentant une face de dessus plate et une face de dessous avec des bords longitudinaux, en étant apte à coopérer avec un appareil impacteur. Il est revendiqué que les bords longitudinaux de la partie commune sont raccordés, d'une part, à la face du dessus de ladite partie commune par un profil largement arrondi et, d'autre part, à la face de dessous par une face inclinée formant un angle obtus par rapport au plan vertical défini par les branches.

Compte tenu des problèmes posés de supprimer toutes aspérités au niveau de la face supérieure de l'agrafe en contact avec la peau, le profil arrondi des bords longitudinaux de la partie commune est constitué par un arc de cercle d'angle au centre de très sensiblement 90°.

Pour résoudre le problème posé d'assurer le blocage angulaire de l'agrafe au niveau de la partie active de l'appareil impacteur, la face inclinée est formée directement dans le prolongement du bord longitudinal arrondi.

Avantageusement, la zone de raccordement entre le bord longitudinal arrondi et la face inclinée est située très sensiblement à la moitié de l'épaisseur de la partie commune.

Pour résoudre le problème posé d'éviter le descellement de l'agrafe, les faces latérales internes et/ou externes des branches et/ou la face de dessous de la partie commune présentent des aspérités d'ancrage.

Pour résoudre le problème posé d'assurer un blocage partait entre l'agrafe et l'appareil impacteur en supprimant tout risque de déviation angulaire de l'agrafe au moment de son impaction, l'appareil impacteur présentant une partie active constituée de deux mors faisant office de pinces en étant assujettis à un organe de manoeuvre formé en bout d'un manche de préhension est remarquable en ce que chacun des mors présente une empreinte en creux correspondant à la section transversale de la partie commune de l'agrafe en vue de son maintien en position au moment de son impaction dans la partie d'os considérée, chaque empreinte en creux présentant une partie de fond plate, une extrémité libre et une face inclinée formée à partir de l'extrémité libre en se raccordant par un large rayon de courbure avec la partie de fond destinée à recevoir la face de dessus de l'agrafe.

Un autre problème que se propose de résoudre l'invention, est de pouvoir impacter l'agrafe de manière complète. Un tel problème résolu en ce que l'extrémité des mors présente un profil externe courbe et effilé pour venir en position de tangence avec la surface de l'os où est impactée l'agrafe, pour que sa face de dessous soit en contact avec ledit os.

L'invention est exposée ci-après plus en détail, à l'aide des dessins annexés dans lesquels :
- la figure 1 est une vue en perspective de l'agrafe selon l'invention.
- la figure 2 est, à une échelle plus importante, une vue en coupe transversale considérée selon la ligne 2.2 de la figure 1.
- la figure 3 est une vue de face de l'agrafe.
- la figure 4 est une vue de face montrant l'agrafe positionnée dans un appareil impacteur.
- la figure 5 est, à une échelle plus importante, une vue en coupe montrant le positionnement de l'agrafe dans la partie de l'appareil impacteur, ainsi que l'impaction de ladite agrafe.

On rappelle, de manière connue, que l'agrafe chirurgicale, désignée dans son ensemble par (1) , est constituée par deux branches parallèles d'ancrage (1a et 1b) réunies d'une manière perpendiculaire par une partie commune rectiligne (1c). L'agrafe a donc une forme de U renversé. L'extrémité des branches (1a) et (1b) est taillée en forme de biseau notamment, pour faciliter leur introduction au niveau de la corticale de l'os. La largeur (l) de la partie commune (1c) est supérieure à l'épaisseur transversale des branches (1a et 1b). Ces dispositions ne sont pas décrites en détail car parfaitement connues pour un homme de métier. De même, les faces latérales internes et externes des branches (1a et 1b), ainsi que la face de dessous de la partie commune (1c), peuvent présenter des aspérités d'ancrage (1d).

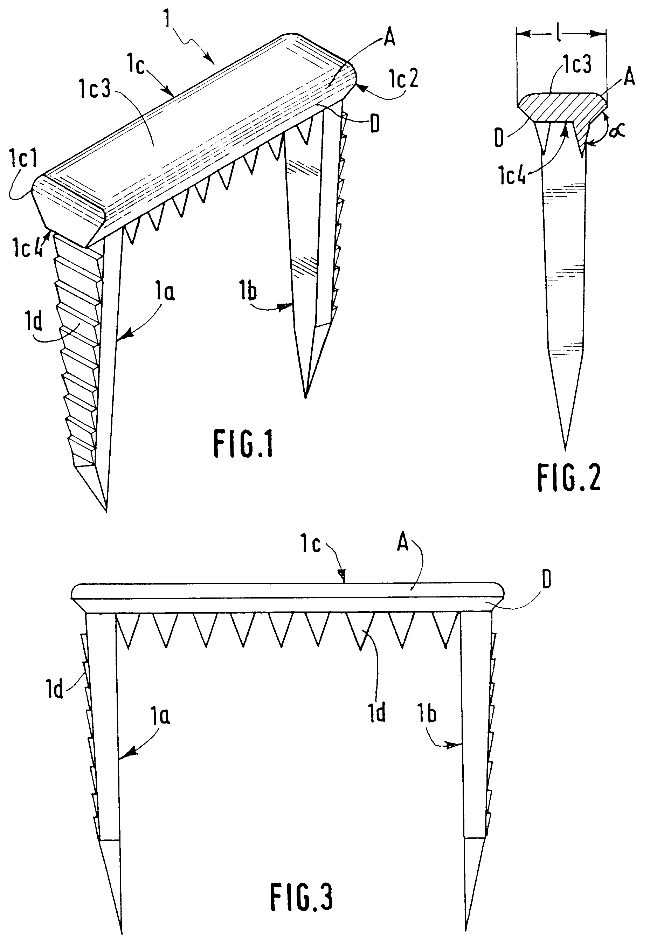

Selon une caractéristique à la base de l'invention, la partie commune (1c) présente une section transversale méplate dont au moins les bords longitudinaux (1c1-1c2) sont raccordés, d'une part, avec la face de dessus (1c3) par un profil largement arrondi (A) et , d'autre part, à la face de dessous (1c4) par une face inclinée (D) (figure 2). Les profils arrondis (A) de chacun des bords longitudinaux (1c1-1c2) sont constitués par un arc de cercle d'angle au centre de très sensiblement 90°. Les faces inclinées (B) sont formées directement dans le prolongement des bords arrondis (A). Ces faces (B) se raccordent avec le plan vertical défini par les branches (1a et 1b) selon un angle obtus (α ).

La zone de raccordement entre le bord arrondi (A) et la face inclinée (B), est située très sensiblement à la moitié de l'épaisseur de la partie commune (1c).

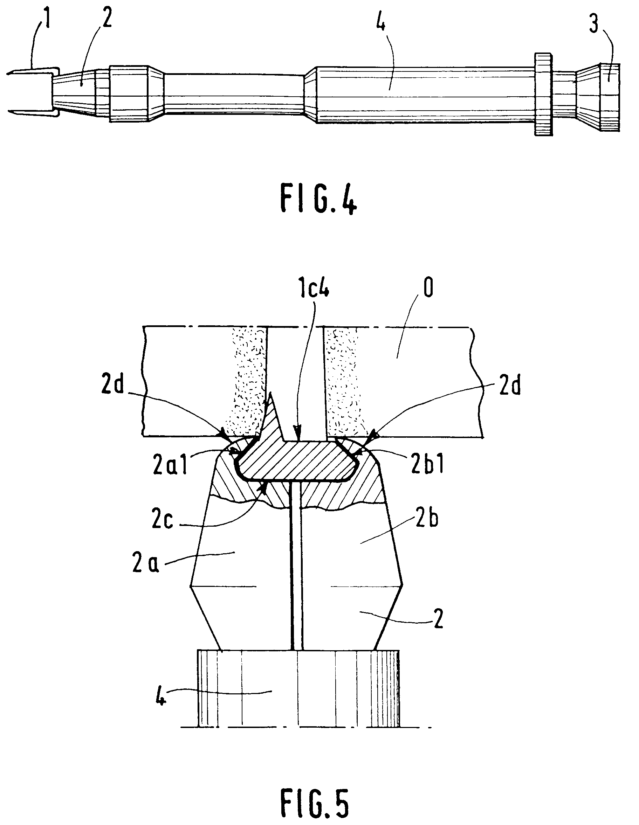

La section transversale ainsi définie de la partie commune (1c) est destinée à coopérer avec la partie active d'un appareil impacteur de tous types connus et appropriés. Comme le montrent les figures 4 et 5, la partie active (2) est constituée par deux mors (2a-2b), faisant office de pinces, en étant assujettis à un organe de manoeuvre (3) formé en bout d'un manche de préhension (4).

Selon l'invention, chacun des mors (2a-2b) présente une empreinte en creux (2a1-2b1) correspondant exactement à la section transversale de la partie commune (1c) de l'agrafe. Notamment chacune de ces empreintes présente une face inclinée formée à partir de l'extrémité libre en se raccordant par un large rayon de courbure de même valeur que les bords arrondis (A) avec la partie de fond (2c) recevant la face de dessus (1c3) de l'agrafe.

Il apparait donc que les faces inclinées des pinces (2a-2b), en combinaison avec les faces inclinées (D) de l'agrafe, assurent le blocage angulaire de ladite agrafe dans la partie active de l'appareil impacteur en évitant tout risque de déviation angulaire au moment de l'impaction.

Suivant une autre caractéristique, et comme le montre la figure 5, l'extrémité des mors (2) présente un profil externe profilé (2d) pour venir en position de tangence avec la surface de l'os (O). Ces dispositions permettent d'impacter en totalité l'agrafe pour que sa face de dessous (1c4) soit en contact avec l'os.

On voit, qu'après avoir impacté l'agrafe, aucune aspérité n'apparait. Les bords externes largement arrondis (A) évitent tout risque de traumatismes lorsque l'agrafe effleure la peau.

Les avantages ressortent bien de la description.

## Revendications

1. Agrafe chirurgicale présentant deux branches parallèles d'ancrage (1a-1b) réunies par une partie commune rectiligne (1c) de section transversale méplate et présentant une face de dessus plate (1c3) et une face de dessous (1c4) avec des bords longitudinaux (1c1-1c2), en étant apte à coopérer avec un appareil impacteur caractérisée en ce que les bords longitudinaux (1c1-1c2) de la partie commune (1c) sont raccordés, d'une part, à la face du dessus (1c3) de ladite partie commune (1c) par un profil largement arrondi (A) et, d'autre part, à la face de dessous (1c4) par une face inclinée (D) formant un angle obtus (α) par rapport au plan vertical défini par les branches (1a) et (1b).

2. Agrafe selon la revendication 1 caractérisée en ce que le profil arrondi (A) de chacun des bords longitudinaux de la partie commune (1c) est constitué par un arc de cercle d'angle au centre de très sensiblement 90°

3. Agrafe selon la revendication 1 caractérisée en ce que la face inclinée (D) est formée directement dans le prolongement du bord longitudinal arrondi (A).

4. Agrafe selon la revendication 3 caractérisée en ce que la zone de raccordement entre le bord longitudinal arrondi (A) et a face inclinée (D) est située très sensiblement à la moitié de l'épaisseur de la partie commune (1c).

5. Agrafe selon l'une quelconque des revendications 1 à 4 caractérisée en ce que les faces latérales internes et/ou externes des branches (1a-1b) et/ou la face de dessous (1c4) de la partie commune (1c) présentent des aspérités d'ancrage (1d).

6. Appareil impacteur présentant une partie active (2) constituée de deux mors (2a-2b) faisant office de pinces en étant assujettis à un organe de manoeuvre (3) formé en bout d'un manche de préhension (4), caractérisé en ce que l'appareil impacteur est destiné à la mise en place d'une agrafe chirurgicale selon l'une des revendications 1 à 5 et que chacun des mors (2a-2b) présente une empreinte en creux (2a1-2b1) correspondant à la section transversale de la partie commune (1c) de l'agrafe en vue de son maintien en position au moment de son impaction dans la partie d'os considérée, chaque empreinte en creux (2a1-2b1) présentant une partie de fond plate (2c), une extrémité libre et une face incliné formée à partir de l'extrémité libre en se raccordant par un large rayon de courbure avec la partie de fond (2c) destinée à recevoir la face de dessus (1c3) de l'agrafe.

7. Appareil selon la revendication 6 caractérisé en ce que l'extrémité des mors (2a-2b) présente un profil externe arrondi et effilé (2d) pour venir en position de tangence avec la surface de l'os où est impactée l'agrafe pour que sa face de dessous soit en contact avec ledit os.

## Claims

1. Surgical staple having two parallel fastening legs (1a-1b) joined by a common straight part (1c) of flattened cross section having a flat upper surface (1c3) and a lower surface (1c4) with longitudinal edges (1c1-1c2) being capable of cooperating with an applicator characterised in that the longitudinal edges (1c1-1c2) of the common part (1c) are joined, on the one hand, to the upper surface (1c3) of said common part (1c) by a gently rounded profile (A) and, on the other hand, to the lower surface (1c4) by a sloping surface (D) forming an obtuse angle (α) relative to the vertical plane defined by legs (1a) and (1b).

2. Staple as claimed in claim 1, characterised in that the rounded profile (A) of each of the longitudinal edges of the common part (1c) consists of an arc of a circle of which the centre angle is essentially 90°.

3. Staple as claimed in claim 1, characterised in that the sloping surface (D) is formed directly as an extension of the rounded longitudinal edge (A).

4. Staple as claimed in claim 3, characterised in that the connection area between the rounded longitudinal edge (A) and the sloping surface (D) is located essentially at half the thickness of common part (1c).

5. Staple as claimed in any of claims 1 to 4, characterised in that the inside and/or outside lateral surfaces of legs (1a-1b) and/or the lower surface (1c4) of the common part (1c) have serrated edges (1d) to improve retention.

6. Applicator having an active part (2) consisting of two jaws (2a-2b) acting as grippers that are automatically controlled by an operating device (3) formed at the end of a handle (4) characterised in that the applicator is intended to place a surgical staple according to one of claims 1 to 5 and in that each of the jaws (2a-2b) has a hollow cavity (2a1-2b1) matching the cross-section of the common part (1c) of the staple so that it can be held in position at the time it is driven into the part of the bone in question, each hollow cavity (2a1-2b1) having a flat bottom part (2c), an open end and a sloping surface formed by the open end that is joined, over a wide radius of curvature, to the bottom part (2c) intended to accommodate the upper surface (1c3) of the staple.

7. Applicator as claimed in claim 6, characterised in that the end of the jaws (2a-2b) has a rounded, tapered profile (2d) that positions itself so that it touches the surface of the bone into which the staple is driven so that its lower surface is in contact with said bone.

## Patentansprüche

1. Chirurgische Klammer mit zwei parallelen Verankerungsschenkeln (1a-1b), die durch einen gemeinsamen geradlinigen Teil (1c) miteinander verbunden sind, dessen Querschnitt abgeflacht ist und der eine flache Oberseite (1c3) und eine Unterseite (1c4) mit Längsrändern (1c1-1c2) aufweist und geeignet ist, mit einem Anschlaggerät zusammenzuwirken, dadurch gekennzeichnet, daß die Längsränder (1c1-1c2) des gemeinsamen Teils (1c) zum einen durch ein leicht abgerundetes Profil (A) mit der Oberseite (1c3) des besagten gemeinsamen Teils (1c) und zum anderen durch eine Schrägfläche (D), die gegenüber der senkrechten, von den Schenkeln (1a) und (1b) beschriebenen Ebene einen stumpfen Winkel (α) bildet, mit der Unterseite (1c4) verbunden sind.

2. Klammer nach Anspruch 1, dadurch gekennzeichnet, daß das abgerundete Profil (A) jedes der Längsränder des gemeinsamen Teils (1c) aus einem Kreisbogen mit einem Winkel in der Mitte von ziemlich genau 90° besteht.

3. Klammer nach Anspruch 1, dadurch gekennzeichnet, daß die Schrägfläche (D) unmittelbar in der Verlängerung des abgerundeten Längsrandes (A) ausgebildet ist.

4. Klammer nach Anspruch 3, dadurch gekennzeichnet, daß sich der Verbindungsbereich zwischen dem abgerundeten Längsrand (A) und der Schrägfläche (D) ziemlich genau in der Materialmitte des gemeinsamen Teils (1c) befindet.

5. Klammer nach Anspruch 1, 2, 3 oder 4, dadurch gekennzeichnet, daß die inneren und/oder äußeren Seitenflächen der Schenkel (1a-1b) und/oder die Unterseite (1c4) des gemeinsamen Teils (1c) verankerungsfördernde Unebenheiten (1d) aufweisen.

6. Anschlaggerät mit einem aktiven Teil (2) bestehend aus zwei zangenartigen, einem Bedienungsorgan (3) am Ende eines Greifschafts (4) untergeordneten Backen (2a-2b), dadurch gekennzeichnet, daß das Anschlaggerät zu dem Einsetzen einer chirurgischen Klammer nach einem der Ansprüche 1-5 bestimmt ist und daß jede der Backen (2a-2b) eine Vertiefung (2a1-2b1) aufweist, die dem Querschnitt des gemeinsamen Teils (1c) der Klammer entspricht, um bei deren Einschlagen in den entsprechenden Knochenteil für einen festen Halt zu sorgen, wobei jede Vertiefung (2a1-2b1) einen flachen Bodenteill (2c), ein freies Ende und eine vom freien Ende ausgehende Schrägfläche aufweist und dabei durch einen breiten Krümmungsradius mit dem Bodenteil (2c) verbunden ist, das zur Aufnahme der Oberseite (1c3) der Klammer bestimmt ist.

7. Gerät nach Anspruch 6, dadurch gekennzeichnet, daß das Endstück der Backen (2a-2b) ein abgerundetes und spitz zulaufendes Außenprofil (2d) aufweist, um sich tangentiell an die Oberfläche des Knochens anzulegen, an der die Klammer eingeschlagen wird, damit die Unterseite mit dem Knochen in Kontakt steht.
